(19) Europäisches Patentamt
European Patent Office
Office européen des brevets

(11) Veröffentlichungsnummer: **0 369 263 A2**

# (12) EUROPÄISCHE PATENTANMELDUNG

(21) Anmeldenummer: **89120425.7**

(51) Int. Cl.[5]: **A61K 31/365, A61K 31/22**

(22) Anmeldetag: **04.11.89**

(30) Priorität: **14.11.88 US 270983**

(43) Veröffentlichungstag der Anmeldung:
**23.05.90 Patentblatt 90/21**

(84) Benannte Vertragsstaaten:
**AT BE CH DE FR GB IT LI LU NL SE**

(71) Anmelder: **F. HOFFMANN-LA ROCHE AG**
**Postfach 3255**
**CH-4002 Basel(CH)**

(72) Erfinder: **McLane, John Arthur**
**24 Howard Street**
**West Haven Connecticut 06516(US)**
Erfinder: **Uskokovic, Milan Radoje**
**253 Highland Avenue**
**Upper Montclair, N.J. 07043(US)**

(74) Vertreter: **Grossner, Lutz, Dr. et al**
**Grenzacher Strasse 124 Postfach 3255**
**CH-4002 Basel(CH)**

## (54) Präparate zur topischen Behandlung von Hautkrankheiten.

(57) Die Verbindungen der Formeln I und II

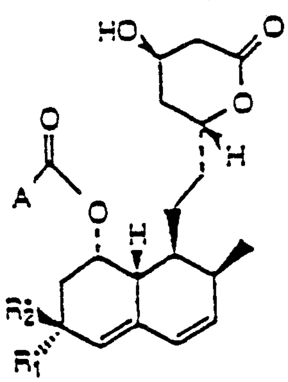

I

und

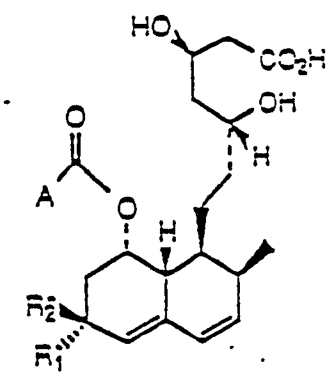

II

worin $R_1$ Wasserstoff und $R_2$ Hydroxy; oder $R_1$ Methyl und $R_2$ Wasserstoff; A Alkyl, Cycloalkyl, Alkenyl, Alkyl substituiert mit Trifluormethyl, Phenyl, Halophenyl, Phenyl-$C_{1-3}$-Alkyl, Phenyl $C_{1-3}$-Alkyl substituiert am Phenyl mit 1-3 Substituenten aus der Gruppe Halo $C_{1-3}$-Alkyl und $C_{1-3}$-Alkoxy ist; oder A
$R_3O(CH_2)_n$-C-$(CH_3)(R_4)$-
ist, worin $R_3$ Wasserstoff oder ($C_{1-5}$-Alkyl)C(O)-; $R_4$ Wasserstoff oder Methyl und n 1-5 ist;
und pharmazeutisch anwendbare Salze, $C_{1-4}$-Alkylester, Acetylamino-$C_{1-4}$-Alkylester, Phenyl-$C_{1-4}$-Alkylester, Dimethylamino-$C_{1-4}$-Alkylester und $\alpha$-Monoglyzeride der Verbindungen der Formel II ,
können zur Behandlung hyperproliferativer Erkrankungen der Haut Verwendung finden.

EP 0 369 263 A2

**Präparate zur topischen Behandlung von Hautkrankheiten**

Die vorliegende Erfindung betrifft die Verwendung von Verbindungen der Formeln

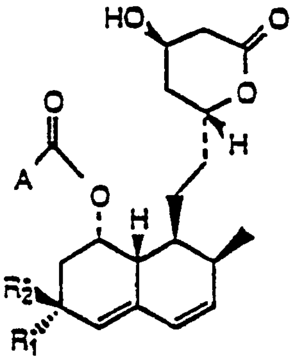

I

und

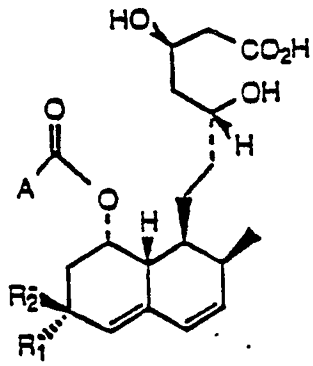

II

worin $R_1$ Wasserstoff und $R_2$ Hydroxy; oder $R_1$ Methyl und $R_2$ Wasserstoff; A Alkyl, Cycloalkyl, Alkenyl, Alkyl substituiert mit Trifluormethyl, Phenyl, Halophenyl, Phenyl-$C_{1-3}$-Alkyl, Phenyl $C_{1-3}$-Alkyl substituiert am Phenyl mit 1-3 Substituenten aus der Gruppe Halo $C_{1-3}$-Alkyl und $C_{1-3}$-Alkoxy ist; oder A
$R_3O(CH_2)_n$-C-$(CH_3)(R_4)$-
ist, worin $R_3$ Wasserstoff oder ($C_{1-5}$-Alkyl)C(O)-; $R_4$ Wasserstoff oder Methyl und n 1-5 ist;
und pharmazeutisch anwendbare Salze, $C_{1-4}$-Alkylester, Acetylamino-$C_{1-4}$-Alkylester, Phenyl-$C_{1-4}$-Alkylester, Dimethylamino-$C_{1-4}$-Alkylester und α-Monoglyzeride der Verbindungen der Formel II.

Bevorzugte Verbindung der Formel I und II sind die Verbindungen der Formeln

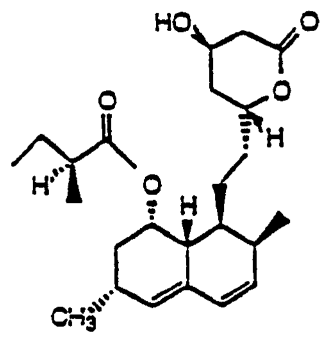

III

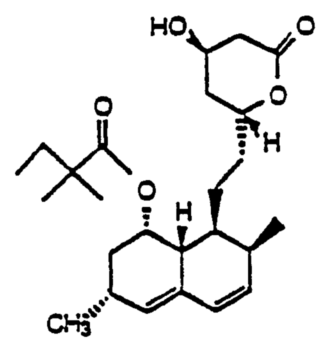

IV

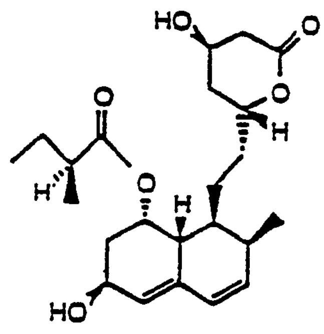

V

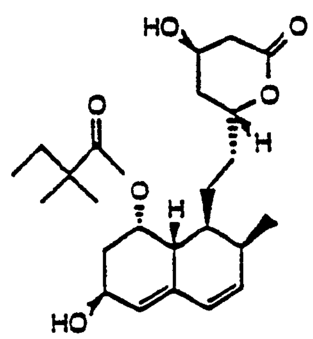

VI

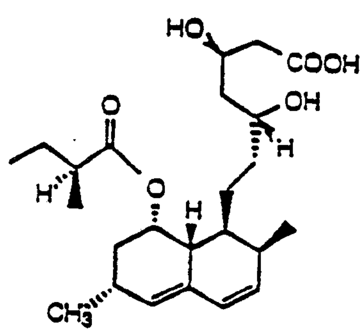

VII

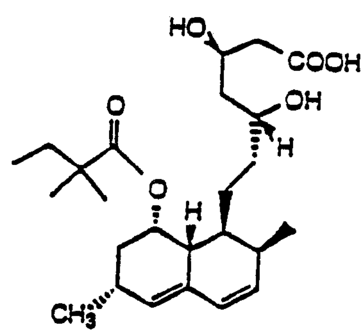

VIII

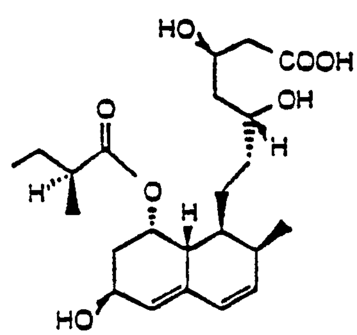

IX

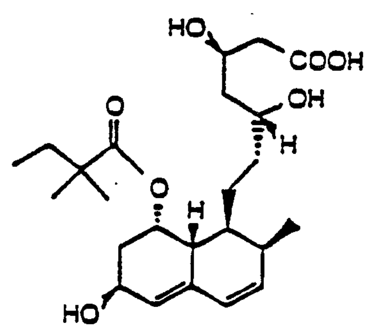

X

Es wurde unerwarteterweise gefunden, dass die Verbindungen der Formel I und II und die oben bezeichneten Ester und Monoglyzeride der Verbindungen der Formel II für die Behandlung hyperproliferativer Hauterkrankungen wie Psoriasis, Basalzellkarzinome, Plattenepithelkarzinome, Keratosis und Keratinisierungsstörungen von Nutzen sind. Diese Verbindungen können oral oder topisch angewandt werden.

Pharmazeutische Präparate zur topischen Anwendung enthaltend eine Verbindung der Formel I oder II oder einen der oben bezeichneten Ester oder Monoglyzeride der Verbindungen der Formel II sind neu und sind ebenfalls Gegenstand der vorliegenden Erfindung.

Die Verbindungen der Formel I und II, worin $R_1$ Methyl und A wie oben beschrieben ist, können nach den in den U.S.-Patentschriften 4,444,784 und 4,450,171 beschriebenen Methoden hergestellt werden.

Verbindungen der Formel I können in Verbindungen der Formel II umgewandelt werden in Analogie zu der in der U.S.-Patentschrift 4,444,784 angegebenen Weise.

Die Verbindungen der Formeln V, VI, IX und X können nach bekannten Methoden hergestellt werden, die in der U.S.-Patentschrift 4,346,227 erörtert sind.

Der hier verwendete Ausdruck "Psoriasis" bezeichnet eine hyperproliferative Hauterkrankung, die den Regulierungsmechanismus der Haut verändert. Insbesondere werden Läsionen gebildet, die primäre und sekundäre Veränderungen der Proliferation in der Epidermis, entzündliche Reaktionen der Haut und die Expression regulatorischer Moleküle wie Lymphokine und Entzündungsfaktoren beinhalten. Psoriatische Haut ist morphologisch durch einen verstärkten Umsatz von Epidermiszellen, verdickte Epidermis, abnormale Keratinisierung entzündlicher Zellinfiltrate in die Dermisschicht und poly morphonucleäre Leukozyteninfiltration in die Epidermis, die eine Zunahme des Basalzellzyklus bedingt, gekennzeichnet. Zusätzlich sind hyperkeratotische und parakeratotische Zellen anwesend.

Der Ausdruck "Keratosis", "Basalzellkarzinome", "Plattenepithelkarzinome" und "Keratinisierungsstörungen" bezieht sich auf hyperproliferative Hauterkrankungen bei denen der Regulierungsmechanismus für die Proliferation und Differenzierung der Hautzellen unterbrochen ist.

Die Verbindungen der Formeln I und II und die oben bezeichneten Salze, Ester und Monoglyceride von Verbindungen der Formel II sind wirksam als Antagonisten der Hauthyperproliferation, d.h. als Mittel, die die Hyperproliferation menschlicher Keratinozyten hemmen. Die Verbindungen antagonisieren ferner Veränderungen in der Differenzierung von Keratinozyten. Die Verbindungen sind infolgedessen als Mittel zur Behandlung hyperproliferativer Hauterkrankungen wie Psoriasis, Basalzellkarzinomen, Keratinisierungsstörungen und Keratosis von Wert.

Der hier verwendete Ausdruck "Halo" bedeutet Chlor, Fluor, Brom oder Jod.

Der hier verwendete Ausdruck "Alkyl" bezeichnet einen geradkettigen oder verzweigten gesättigten Kohlenwasserstoffrest mit 1-10 C-Atomen wie Methyl, Aethyl, Propyl, Isopropyl.

Der Ausdruck "Cycloalkyl" bezeichnet einen Cycloalkylrest mit 3-10 C-Atomen wie Cyclopropyl und Cyclobutyl.

Der Ausdruck "Alkenyl" bezeichnet einen geradkettigen oder verzweigten Alkenylrest mit 2-10 C-Atomen wie Vinyl und Propenyl.

Der Ausdruck "Alkyl substituiert mit Trifluormethyl" bezeichnet einen geradkettigen oder verzweigten $C_{1-10}$-Alkylrest an dem ein Wasserstoff durch Trifluormethyl ersetzt ist.

Der Ausdruck "Halophenyl" bezeichnet Phenyl, das mit bis zu 3 Halogenen substituiert ist.

Der Ausdruck "Phenyl-$C_{1-3}$-Alkyl" bezeichnet einen Alkylrest mit 1-3 C-Atomen, von dem eines der Wasserstoffe durch eine Phenylgruppe ersetzt ist.

In den hier dargestellten Formeln bezeichnet eine keilförmig verdickte Linie (◀) einen Substituenten, der oberhalb der Ebene des Moleküls liegt ($\beta$-Stellung) eine unterbrochene Linie (ıııı) oder (---) einen Substituenten der unterhalb der Molekülebene liegt ($\alpha$-Stellung).

Besonders bevorzugte Verbindungen der Formel I ist die Verbindung der Forml III, die im folgenden als Mevinolin bezeichnet wird und die Verbindung der Formel IV, die im folgenden als Synvinolin bezeichnet wird.

Die Verbindungen der Formel III und IV sind bekannt und können nach bekannten Methoden, beispielsweise wie in der U.S.-Patentschrift 4,346,227 beschrieben, hergestellt werden.

Die Verbindungen der Formel I und II und die oben bezeichneten Salze, Ester und Monoglyceride der Verbindungen der Formel II können, wie oben beschrieben, oral verabreicht werden zur Behandlung hyperproliferativer Hauterkrankungen wie Psoriasis, Basalzellkarzinomen, Keratinisierungsstörungen und Keratosis an Warmblüter, die einer solchen Behandlung bedürfen. Insbesondere können die Verbindungen der Formel I wie oben beschrieben, oral an Erwachsenen in Dosierungen im Bereich von etwa 10 bis etwa 80 mg pro Tag vorzugsweise 10-50 mg pro Tag zur Behandlung hyperproli ferativer Hauterkrankungen, wie Psoriasis, Basalzellkarzinomen, Plattenepithelkarzinomen, Keratinisierungsstorungen und Keratosis verabreicht werden.

Die Verbindungen der Formel I und II können auch topisch wie oben beschrieben angewandt werden. Insbesondere können die Verbindungen topisch in Dosierungen die etwa 100-200 Microgramm pro Gramm Formulierung erhalten pro Tag bei solchen Erkrankungen verabreicht werden, insbesondere in einer Menge von 1-50 Microgramm pro Gramm topische Formulierung pro Tag.

Die Wirksamkeit der erfindungsgemässen Verbindungen als Mittel zur Behandlung hyperproliferativer Hauterkrankungen lässt sich durch die Fähigkeit der Verbindungen zeigen, die Proliferation von Keratinozyten in Zellkulturen von Keratinozyten aus Vorhäuten Neugeborener zu hemmen. Die Resultate sind in Tabelle I dargestellt.

TABELLE I

| HEMMWIRKUNG DER TESTVERBINDUNGEN AUF DIE KERATINOZYTENPROLIFERATION | | | |
|---|---|---|---|
| Verbindung | Dosierung der Verbind. (M) | %-Hemmung der Keratinocyten-Proliferation | Standardabweichung |
| 1. Aethanol (Kontrolle) | | 0.00 | 24.48 |
| 2. Mevinolin | $10^{-10}$ | 23.2 | 26.7 |
| | $10^{-8}$ | 28.9 | 17.57 |
| | $10^{-7}$ | 38.6 | 12.82 |
| | $10^{-6}$ | 84.21 | 14.51 |
| 3. Synvinolin | $10^{-10}$ | 0.00 | 24.08 |
| | $10^{-8}$ | 11.17 | 26.42 |
| | $10^{-7}$ | 33.60 | 26.34 |
| | $10^{-6}$ | 62.06 | 24.31 |

Jede Verbindung wird dreifach getestet bei jeder Konzentration.

Die vorstehenden Resultate zeigen, dass Verbindungen der Formel I bei einer Dosierung von $10^{-6}$M die Keratinozytenproliferation in einem Ausmass von mehr als 50% hemmen ohne zelltoxisch zu sein. Beispielsweise hemmt Mevinolin bei dieser Dosierung 84,21% der Keratinozytenproliferation und Synvinolin hat einen Hemmeffekt von 62,06% auf die Keratinozytenproliferation bei dieser Dosierung.

Diese Zahlen zeigen, dass die Verbindungen der Formel I die Proliferation menschlicher Keratinozyten in vitro ohne Zelltoxizität vermindern. Aus diesen Resultaten ist zu sehen, dass jede der getesteten Verbindung als Mittel zur Behandlung hypoproliferativer Hauterkrankungen wie Psoriasis von Wert ist.

Zur Herstellung oraler Dosierungsformen können die Verbindungen der Formel I und II und die oben bezeichneten Ester und Monoglyzeride der Verbindungen der Formel II in Kapseln, Tabletten und ähnlichem mit pharmazeutisch verträglichen Trägermaterialien verarbeitet werden.

Beispiele von pharmazeutisch anwendbaren Trägermaterialien die in Kapseln eingearbeitet werden können. sind die folgenden: Bindemittel wie Tragacanthgummi, Akaziengummi, Maisstärke oder Gelatine, Excipientien, wie Dicalciumphosphat, Sprengmittel, wie Maisstärke, Kartoffelstärke und Algensäure; Schmiermittel wie Magnesiumstearat, Süsstoffe, wie Sucrose, Laktose oder Saccharin, Geschmacksstoffe, wie Pfefferminz, Wintergrünöl oder Kirschöl. Verschiedene andere Stoffe können als Ueberzugsmaterialien vorhanden sein oder in anderer Form um die physikalische Form der Dosiseinheit zu modifizieren. Beispielsweise können Tabletten mit Shellac oder Zucker oder beidem überzogen sein. Ein Sirup oder Elixir kann die wirksame Verbindung, Sucrose als Süssmittel, Methyl und Propylparaben als Konservierungsstoffe, einen Farbstoff und ein Geschmacksstoff wie Kirsch- oder Orangengeschmack enthalten.

Eine bevorzugte Formulierung für eine orale Dosierungsform der Verbindung der Formel I und II in Kapselform wird in Beispiel 1 nachstehend angegeben.

## Beispiel 1

Eine Formulierung für die orale Verabreichung kann folgende Zusammensetzung haben

| | |
|---|---|
| 1. Verbindung der Formel I or II | 20 Milligram |
| 2. Lactose | 150 Milligram |
| 3. Stärke 1500 | 30 Milligram |
| 4. Talk | 20 Milligram |

Bestandteil 1 wird mit einem Teil 2 gemischt, danach werden 3 und 4 zugesetzt und gemischt. Schliesslich wird der Rest des Bestandteils 2 zugesetzt, gründlich gemischt und die Masse durch eine geeignete Mühle gegeben. Das so erhaltene Präparat wird in Kapseln abgefüllt.

Topische Formulierungen umfassen Gele, Crèmes, Lotionen, Salben, Puder, Aerosole und andere herkömmliche Formulierungen zur Anwendung von Heilmitteln auf die Haut. Neben der Anwendung auf die Haut können die topischen Formulierungen der vorliegenden Erfindung auch angewandt werden bei der Behandlung von Entzündung von Schleimhäuten die der topischen Behandlung zugänglich sind. Beispielsweise können die topischen Formulierungen auf die Schleimhäute des Mundes oder des unteren Colons aufgebracht werden.

Beispiel 2

Eine Formulierung für die topische Verabreichung der Verbindungen der Formel I und II kann folgende Zusammensetzung aufweisen

| | |
|---|---|
| 1. Verbindung der Formel I oder II | 10,0 Mikrogramm |
| 2. Stearylalkohol | 4,0 g |
| 3. Cetylalkohol | 4,0 g |
| 4. Mineralöl | 3,0 g |
| 5. Polysorbat 60 | 4,5 g |
| 6. Sorbitanstearat | 4,5 g |
| 7. Propylenglycol | 10,0 g |
| 8. Methylparaben | 0,18 g |
| 9. Propylparaben | 0,02 g |
| 10. Wasser | q.s. ad 100,00 g |

Bestandteile 2-6 werden auf 80° erwärmt bis alles geschmolzen ist. Danach wird Bestandteil 1 in der öligen Phase gelöst. Bestandteil 7 und 10 werden auf 90° erwärmt und die Bestandteile 8 und 9 werden in der so erhaltenen wässrigen Phase gelöst. Danach wird die wässrige Phase zur Oelphase gegeben und rasch gerührt, so dass eine Emulsion erhalten wird. Danach wird langsam auf 50° abkühlen gelassen um die Emulsion zu verfestigen. Unter weiterem Rühren wird das Präparat auf Raumtemperatur abgekühlt.

**Ansprüche**

1. Pharmazeutisches Präparat zur topischen Verabreichung, enthaltend eine Verbindung der Formeln

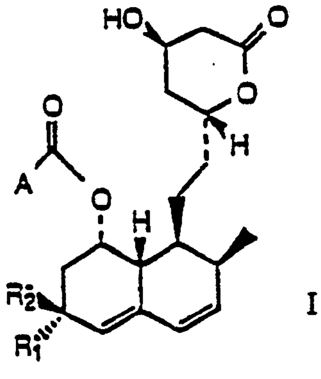 I und 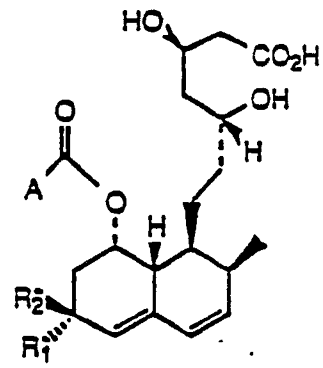 II

worin $R_1$ Wasserstoff und $R_2$ Hydroxy; oder $R_1$ Methyl und $R_2$ Wasserstoff; A Alkyl, Cycloalkyl, Alkenyl, Alkyl substituiert mit Trifluormethyl, Phenyl, Halophenyl, Phenyl-$C_{1-3}$-Alkyl, Phenyl $C_{1-3}$-Alkyl substituiert am Phenyl mit 1-3 Substituenten aus der Gruppe Halo $C_{1-3}$-Alkyl und $C_{1-3}$-Alkoxy ist; oder A
$R_3O(CH_2)_n$-C-$(CH_3)(R_4)$-
ist, worin $R_3$ Wasserstoff oder ($C_{1-5}$-Alkyl)C(O)-; $R_4$ Wasserstoff oder Methyl und n 1-5 ist; und pharmazeutisch anwendbare Salze, $C_{1-4}$-Alkylester, Acetylamino-$C_{1-4}$-Alkylester, Phenyl-$C_{1-4}$-Alkylester, Dimethylamino-$C_{1-4}$-Alkylester und α-Monoglyzeride der Verbindungen der Formel II.

2. Präparat gemäss Anspruch 1, worin in der Verbindung der Formel I oder II A Alkyl ist.

3. Präparat gemäss Anspruch 2, enthaltend Mevinolin oder Synvinolin.

4. Präparat gemäss den Ansprüchen 1-3, enthaltend etwa 1 bis etwa 200 µg einer Verbindung der Formeln I oder II oder ein pharmazeutisch anwendbares Salz, $C_{1-4}$-Alkylester, Acetylamino-$C_{1-4}$-Alkylester, Phenyl-$C_{1-4}$-Alkylester, Dimethylamino-$C_{1-4}$-Alkylester oder α-Monoglyzerid der Verbindung der Formel II.

5. Verwendung einer Verbindung der Formel I oder II oder eines pharmazeutisch anwendbaren Salzes, $C_{1-4}$-Alkylesters, Acetylamino-$C_{1-4}$-Alkylester, Phenyl-$C_{1-4}$-Alkylester, Dimethylamino-$C_{1-4}$-Alkylester oder α-Monoglyzerid der Verbindung der Formel II zur Herstellung eines topischen Präparats zur Behandlung hyperproliferativer Hauterkrankungen.

6. Verwendung einer Verbindung der Formel I oder II, in der A Alkyl ist, oder eines pharmazeutisch anwendbaren Salzes, $C_{1-4}$-Alkylesters, Acetylamino-$C_{1-4}$-Alkylester, Phenyl-$C_{1-4}$-Alkylester, Dimethylamino-$C_{1-4}$-Alkylester oder α-Monoglyzerid der Verbindung der Formel II gemäss Anspruch 5.

7. Verwendung von Mevinolin oder Synvinolin gemäss Anspruch 5.

8. Verwendung gemäss den Ansprüchen 5-7 zur Herstellung von Präparaten, die etwa 1 bis etwa 200 µg einer Verbindung der Formel I oder II oder eines pharmazeutisch anwendbaren Salzes, $C_{1-4}$-Alkylesters, Acetylamino-$C_{1-4}$-Alkylester, Phenyl-$C_{1-4}$-Alkylester, Dimethylamino-$C_{1-4}$-Alkylester oder α-Monoglyzerid der Verbindung der Formel II enthalten.